# EUROPEAN PATENT APPLICATION

(11) **EP 3 957 719 A1**
(43) Date of publication of application: **23.02.2022**
(21) Application number: 21192325.5
(22) Date of filing: 20.08.2021
(51) Int. Cl.: C12N 5/0775, A61K 35/28, A61P 3/08, A61P 1/16, A61P 1/18

(54) **MODIFIED MESENCHYMAL STEM CELL CULTURE MEDIUM, BONE MARROW MESENCHYMAL STEM CELLS AND CULTURE METHODS AND USE THEREOF**

(30) Priority: 21.08.2020 CN 202010847844
(71) Applicant: Shenzhen Hongji Biotechnology Co., Ltd, Shenzhen, Guangdong 518063 (CN)
(72) Inventor: JI, Guangju, Shenzhen (CN); YANG, Zhiguang, Shenzhen (CN); LI, Jing, Shenzhen (CN)
(74) Representative: Bassil, Nicholas Charles

(57) **Abstract**

The present disclosure relates to a modified mesenchymal stem cell culture medium, a bone marrow mesenchymal stem cell and a culture method and use thereof, the modified mesenchymal stem cell culture medium comprising a basal culture medium, a first component, and melatonin, wherein the first component is at least one selected from the group consisting of coenzyme Q10 and mitoquinone mesylate, and has a working concentration in a range from 1 µM to 20 µM, the melatonin has a working concentration in a range from 1 µM to 20 µM, and the first component and the melatonin are in a molar ratio ranging from 1:0.2 to 1:10. The modified mesenchymal stem cell culture medium can increase the mitochondrial activity and the number of mitochondria in mesenchymal stem cells.

## Description

### TECHNICAL FIELD

The present disclosure relates to cell culture technology, in particular to a modified mesenchymal stem cell culture medium, a bone marrow mesenchymal stem cell, and a culture method and use thereof.

### BACKGROUND

Mitochondria are important intracellular semi-autonomous organelles that contain mitochondrial DNA capable of partially encoding mitochondrial genetic information and are the main place for aerobic respiration in the cell, responsible for providing energy to the cell. In addition, mitochondria play an important role in cell differentiation, cell secretion, cell proliferation, and apoptosis.

Mesenchymal stem cells (MSCs), which are kinds of pluripotent stem cells derived from the mesoderm and mainly exist in connective tissue and the interstitium of organs, possess strong proliferation and differentiation capabilities and low immunogenicity, thus having broad application prospects. Bone marrow mesenchymal stem cells (BMSCs) are of the current mesenchymal stem cells most used. The use of bone marrow mesenchymal stem cells in treatment of a disease mainly relies on the following mechanisms: 1) replacement of cells: the bone marrow mesenchymal stem cells can differentiate into a variety of cells to supplement the damage or loss of cells in the body, for example, the bone marrow mesenchymal stem cells can differentiate into muscle cells, hepatocytes, osteoblasts, adipocytes, chondrocytes, stromal cells, etc, in a suitable in vivo or in vitro environment; 2) secretion by cells: the bone marrow mesenchymal stem cells can secrete a variety of cytokines, extracellular matrix, oxidoreductases, heat-activated proteins, and other active small molecules to participate metabolism in the body; and 3) mitochondrial transfer: the mitochondria of the bone marrow mesenchymal stem cells can be transferred to damaged and senescent tissues or cells to repair or activate the damaged and senescent tissues and cells and to regulate cell metabolism.

However, mitochondrial DNA in the mesenchymal stem cells is easily attacked and damaged due to the high-level reactive oxygen species in the environment of mitochondria in the mesenchymal stem cells. With the subculture of mesenchymal stem cells, their mitochondria are liable to decrease activity and reduce numbers.

### SUMMARY

Accordingly, it is necessary to provide a modified mesenchymal stem cell culture medium that can delay the decrease of mitochondrial activity in mesenchymal stem cells and increase the number of mitochondria. In addition, a bone marrow mesenchymal stem cell with high mitochondrial activity and a large number of mitochondria, and a culture method and use thereof are also provided.

The present disclosure provides a modified mesenchymal stem cell culture medium, comprising a basal culture medium, a first component, and melatonin, wherein the first component is at least one selected from the group consisting of coenzyme Q10 and mitoquinone mesylate, and has a working concentration in a range from 1 µM to 20 µM, the melatonin has a working concentration in a range from 1 µM to 20 µM, and the first component and the melatonin are in a molar ratio ranging from 1:0.2 to 1:10.

The modified mesenchymal stem cell culture medium, by adding at least one of coenzyme Q10 and mitoquinone mesylate and the melatonin in a molar ratio ranging from 1:0.2 to 1:10 into the basal culture medium, makes the mitochondrial DNA of mesenchymal stem cells less likely to be damaged by the attack of reactive oxygen species, can delay the reduction of mitochondrial activity during the culture of mesenchymal stem cells, and can increase the number of mitochondria in mesenchymal stem cells.

In an embodiment, the modified mesenchymal stem cell culture medium further includes a second component, and the second component is at least one selected from the group consisting of nicotinamide mononucleotide, nicotinamide ribose, and NADH.

In an embodiment, the first component and the second component are in a molar ratio ranging from 1:0.2 to 1:10.

In an embodiment, the modified mesenchymal stem cell culture medium further comprises a second component; the second component is nicotinamide mononucleotide, and the first component is coenzyme Q10; and the working concentration of the first component is 1 µM to 5 µM, the working concentration of the melatonin is 1 µM to 5 µM, and the working concentration of the second component is 0.5 µM to 10 µM.

In an embodiment, the basal culture medium is one selected from DMEM, EMEM, IMDM, GMEM, RPMI-1640, and α-MEM.

In an embodiment, the modified mesenchymal stem cell culture medium further comprises at least one of vitamin B, minerals, polyphenols, L-carnitine, alpha lipoic acid, pyrroloquinoline quinone, and creatine.

The present disclosure provides a method for culturing bone marrow mesenchymal stem cells comprising the following step:
culturing the bone marrow mesenchymal stem cells in the modified mesenchymal stem cell culture medium as described above.

In an embodiment, the bone marrow mesenchymal stem cells are human bone marrow mesenchymal stem cells.

The present disclosure provides a bone marrow mesenchymal stem cell obtained by the method for culturing bone marrow mesenchymal stem cells as described above.

The present disclosure provides use of the bone marrow mesenchymal stem cells as described above in the preparation of a medicament for the treatment of type 2 diabetes or fatty liver.

The present disclosure provides a medicament for the treatment of type 2 diabetes or fatty liver comprising the bone marrow mesenchymal stem cells as described above.

### BRIEF DESCRIPTION OF THE DRAWINGS

FIG. 1 shows a relation of body weight of mice versus time in HFD control, BMSC, and eBMSC groups in Example 3.
FIG. 2 shows results of glucose tolerance test at week 36 in mice in HFD control, BMSC, and eBMSC groups in Example 3.
FIG. 3 shows the paraffin sections in Example 4.

### DETAILED DESCRIPTION OF THE EMBODIMENTS

In order to facilitate the understanding of the present disclosure, the present disclosure will be described below in a more comprehensive manner. The present disclosure may, however, be embodied in many different forms and should not be construed as limited to the embodiments set forth herein. Rather, these embodiments are provided so that this disclosure will be thorough and complete.

All technical and scientific terms used herein have the same meaning as commonly understood by those skilled in the technical field of the present disclosure unless otherwise defined. The terms used in the description of the present disclosure herein are for the purpose of describing particular embodiments only and are not intended to limit the present disclosure.

An embodiment of the present disclosure provides a modified mesenchymal stem cell culture medium, comprising a basal culture medium, a first component, and melatonin, wherein the first component is at least one selected from the group consisting of coenzyme Q10 and mitoquinone mesylate, and has a working concentration in a range from 1 µM to 20 µM, the melatonin has a working concentration in a range from 1 µM to 20 µM, and the first component and the melatonin are in a molar ratio ranging from 1:0.2 to 1:10. The modified mesenchymal stem cell culture medium can delay the decrease of mitochondrial activity in mesenchymal stem cells and increase the number of mitochondria. The mesenchymal stem cells cultured in this culture medium have high mitochondrial activity and a large number of mitochondria.

Specifically, the basal culture medium is used to provide essential nutrients for the growth of the mesenchymal stem cells. The basal culture medium in the present disclosure is one selected from DMEM, EMEM, IMDM, GMEM, RPMI-1640, and α-MEM. The basic culture medium may be a serum-free mesenchymal stem cell culture medium, or a serum-containing mesenchymal stem cell culture medium.

In an embodiment, the modified mesenchymal stem cell culture medium contains an additive. Specifically, the additive may be well-known additives as long as they do not inhibit the proliferation of the mesenchymal stem cells. There are, for example, growth factors (such as insulin, etc.), iron sources (such as transferrin, etc.), polyamines (such as putrescine, etc.), minerals (such as sodium selenate, etc.), sugars (such as glucose, etc.), organic acids (such as pyruvic acid, lactic acid, etc.), or the like.

In an embodiment, the first component is coenzyme Q10. In an embodiment, the first component is mitoquinone mesylate (mitoQ for short).

In an embodiment, the working concentration of the first component is in a range from 1 µM to 20 µM. The first component at the working concentration in a range from 1 µM to 20 µM may protect the mitochondira, increase the mitochondrial activity, and reduce ROS. Further, the working concentration of the first component is in a range from 1 µM to 10 µM. Still further, the working concentration of the first component is in a range from 1 µM to 5 µM.

In an embodiment, the working concentration of the coenzyme Q10 is in a range from 1 µM to 20 µM. The coenzyme Q10 at the working concentration in a range from 1 µM to 20 µM may protect the mitochondira, increase the mitochondrial activity, and reduce ROS. Further, the working concentration of the coenzyme Q10 is in a range from 1 µM to 10 µM. Still further, the working concentration of the coenzyme Q10 is in a range from 1 µM to 5 µM.

In an embodiment, the first component and the melatonin are in a molar ratio ranging from 1:0.2 to 1:10. The first component and the melatonin in a molar ratio ranging from 1:0.2 to 1:10 may promote the proliferation of mesenchymal stem cells, enhance the cell viability, increase the mitochondrial activity, and reduce ROS. Further, the first component and the melatonin are in a molar ratio ranging from 1:1 to 1:3.

In an embodiment, the first component is coenzyme Q10, and the first component and the melatonin are in a molar ratio ranging from 1:0.2 to 1:10, and further, in a molar ratio ranging from 1:1 to 1:3.

In an embodiment, the working concentration of the melatonin is in a range from 1 µM to 20 µM. Further, the working concentration of the melatonin is in a range from 1 µM to 10 µM. Still further, the working concentration of the melatonin Q10 is in a range from 1 µM to 5 µM.

In an embodiment, the modified mesenchymal stem cell culture medium further comprises a second component, and the second component is at least one selected from the group consisting of nicotinamide mononucleotide (NMN), nicotinamide ribose (NR), and NADH. The second component functions together with the first component and melatonin to further increase the mitochondrial activity and the number of mitochondria in mesenchymal stem cells. Further, the first component and the second component are in a molar ratio ranging from 1:0.2 to 1:10. When the first component and the second component are in a molar ratio ranging from 1:0.2 to 1:10, the mitochondrial activity can be further increased. Still further, the first component and the second component are in a molar ratio ranging from 1:0.2 to 1:2.5.

In an embodiment, the second component is the nicotinamide mononucleotide, and the nicotinamide mononucleotide has a working concentration in a range from 0.5 µM to 10 µM. Further, the working concentration of the nicotinamide mononucleotide is in a range from 0.5 µM to 5 µM.

In an embodiment, the first component is coenzyme Q10, the coenzyme Q10 has a working concentration in a range from 1 µM to 5 µM, the working concentration of the melatonin isin a range from 1 µM to 5 µM, and the working concentration of the nicotinamide mononucleotide is in a range from 0.5 µM to 10 µM. Further, the coenzyme Q10 and the melatonin are in a molar ratio from 1:1 to 1:3; the coenzyme Q10 and the nicotinamide mononucleotide are in a molar ratio from 1:0.2 to 1:2.5.

In an embodiment, the modified mesenchymal stem cell culture medium is composed of a basal culture medium, a serum substitute, EGF, BEGF, coenzyme Q10, melatonin, and nicotinamide mononucleotide; wherein the working concentration of the coenzyme Q10 is in a range from 1 µM to 5 µM, the working concentration of the melatonin is in a range from 1 µM to 5 µM, and the working concentration of the nicotinamide mononucleotide is in a range from 0.5 µM to 10 µM. Further, the coenzyme Q10 and the melatonin are in a molar ratio from 1:1 to 1: 3; the coenzyme Q10 and the nicotinamide mononucleotide are in a molar ratio from 1:0.2 to 1:2.5.

In an embodiment, the modified mesenchymal stem cell culture medium further comprises at least one of vitamin B, minerals (or inorganic salt), polyphenols, L-carnitine, alpha lipoic acid, pyrroloquinoline quinone, and creatine.

It should be noted that the working concentration of the first component refers to a concentration of the first component in the modified mesenchymal stem cell culture medium; the working concentration of melatonin refers to a concentration of the melatonin in the modified mesenchymal stem cell culture medium; and the working concentration of the second component refers to a concentration of the second component in the modified mesenchymal stem cell culture medium.

In an embodiment, the modified mesenchymal stem cell culture medium as described above is used to culture the bone marrow mesenchymal stem cells. In an embodiment, the modified mesenchymal stem cell culture medium as described above can also be used to culture other mesenchymal stem cells, for example, umbilical cord mesenchymal stem cells, other than the bone marrow mesenchymal stem cells, and may increase the activity and number of mitochondria of other mesenchymal stem cells.

An embodiment of the present disclosure also provides a method for culturing the bone marrow mesenchymal stem cells, comprising step a) and b), specifically,
step a): isolating bone marrow mesenchymal stem cells from bone marrow.

Specifically, the method for isolating bone marrow mesenchymal stem cells from bone marrow is a whole bone marrow culture method (also called a direct culture method) or a density gradient centrifugation method.

In an embodiment, the bone marrow mesenchymal stem cells are isolated from the bone marrow by the density gradient centrifugation method, specifically, by adding normal saline in an equal volume to the bone marrow for dilution, followed by the diluted bone marrow slowly onto a surface of lymphocyte isolation liquid (Ficoll isolation liquid) in a ratio of 1 part by volume of Ficoll isolation liquid to 2 parts by volume of the diluted bone marrow, centrifuging the resultant mixture in a horizontal centrifuge at 2000 r/min at 20°C for 25-30 minutes, and removing the bone marrow mesenchymal stem cells from the interface. Then, the bone marrow mesenchymal stem cells are washed with normal saline 2 to 3 times and counted after adding an appropriate amount of culture medium.

In this embodiment, the bone marrow is derived from human. It is understood that in some other embodiments, the bone marrow may also be animal bone marrow, such as mouse bone marrow. In some embodiments, step a) may be omitted, and the bone marrow mesenchymal stem cells may be purchased.

step b): culturing the bone marrow mesenchymal stem cells in any of the modified mesenchymal stem cell culture mediums as described above.

Specifically, the bone marrow mesenchymal stem cells isolated from the bone marrow are primarily cultured in any of the modified mesenchymal stem cell culture mediums as described above.

In an embodiment, the step of primarily culturing the bone marrow mesenchymal stem cells includes: seeding the bone marrow mesenchymal stem cells isolated from bone marrow at a concentration of 5×10⁶ cells/mL in any of the modified mesenchymal stem cell culture medium as described above for culture, refreshing the medium after 3 days to remove non-adherent cells, and refreshing half of the spent medium every 3 to 4 days. It should be noted that the primary culture herein refers to the culture during the period from seeding the cells obtained from the tissue taken out from human's body to the first subculture.

In an embodiment, a subculture step is also included after the step of primarily culturing the bone marrow mesenchymal stem cells. Specifically, the subculture step is performed when the cells reach 90% confluency. Further, the subculture step includes: digesting the bone marrow mesenchymal stem cells, and seeding the digested bone marrow mesenchymal stem cells at a density of 2×10⁵ cells/mL to 5×10⁵ cells/mL in a fresh modified mesenchymal stem cell medium for culture.

In an optional specific example, once the cells reach 90% confluence, the spent medium in the flask is discarded, and the bone marrow mesenchymal stem cells are washed twice with normal saline. 0.25% (2.5g/L, 0.25%) trypsin preheated at 37°C (containing 1mmol/L EDTA, that is, prepared with 1mmol/L EDTA-Na2) is added for digestion for 1-2 min. The cells are observed under an inverted microscope. Once the bone marrow mesenchymal stem cells shrink, the modified mesenchymal stem cell culture medium (approximately 3 mL) was added to stop the digestion by trypsin. After repeatedly pipetting up and down, the cells are collected in a 15mL centrifuge tube and then centrifuged at 1500r/min for 10 min. After the supernatant was discarded, the cells are washed twice with normal saline solution to thoroughly wash away the remaining trypsin. The bone marrow mesenchymal stem cells are resuspended in the modified mesenchymal stem cell culture medium, and seeded at 2×10⁵ cells/mL-5×10⁵ cells/mL for subculture in a new flask.

The method for culturing bone marrow mesenchymal stem cells as described above is simple and easy to operate. It has been confirmed that the bone marrow mesenchymal stem cells cultured according to the above method have high mitochondrial activity and a large number of mitochondria.

In addition, an embodiment of the present disclosure provides a bone marrow mesenchymal stem cell obtained by the method for culturing bone marrow mesenchymal stem cells as described above.

An embodiment of the present disclosure provides use of the bone marrow mesenchymal stem cells as described above in the preparation of a medicament for the treatment of type 2 diabetes or fatty liver. The bone marrow mesenchymal stem cells as described above have high mitochondrial activity and a large number of mitochondria. It has been confirmed that the bone marrow mesenchymal stem cells as described above have significant therapeutic effects on both type 2 diabetes and fatty liver.

An embodiment of the present disclosure also provides a medicament, which can be used for the treatment of type 2 diabetes or fatty liver. Specifically, the medicament comprises the above-mentioned bone marrow mesenchymal stem cells as an active ingredient. In some embodiments, the above-mentioned medicament also comprises a pharmaceutically acceptable excipient, for example, a carrier for preserving the above-mentioned bone marrow mesenchymal stem cells.

### Examples

A detailed description will be given below in conjunction with specific examples. The following examples do not include other components except unavoidable impurities unless specifically stated. The agents and instruments used in the examples are all common in the art unless specifically stated. In the examples, the experimental methods that are not specified with specific conditions are implemented in accordance with conventional conditions, such as the conditions described in the literature, books, or the approach recommended by the manufacturer. Herein, "µM" refers to "µmol/L"; and "mM" refers to "mmol/L". In the following examples, coenzyme Q10 was purchased from Selleck, #S2398; NMN was purchased from Selleck, #S5259; and melatonin was purchased from Sigma, #73-31-4. A serum-free mesenchymal stem cell culture medium was prepared by adding a serum substitute (purchased from Helios), EGF (purchased from Sigma), and BFGF (purchased from Sigma) into α-MEM (purchased from Sigma). In the serum-free mesenchymal stem cell medium, α-MEM and serum substitute were in a volume ratio of 20:1, the concentration of EGF was 1µg/L, and the concentration of BEGF was 1µg/L.

### Example 1

(1) Isolation of bone marrow mesenchymal stem cells: 15 mL of human bone marrow was extracted and treated with 100 U heparin per milliliter of bone marrow for anticoagulation. The normal saline was added in an equal volume to and blended with the bone marrow, and then the diluted bone marrow was slowly added onto the surface of the lymphocyte isolation liquid (Ficoll isolation liquid) in a ratio of 1 part of Ficoll isolation liquid to 2 parts of the diluted bone marrow. The resultant mixture was centrifuged in a horizontal centrifuge at 2000 r/min at 20°C for 30 minutes, and the bone marrow mesenchymal stem cells removed from the interface were washed with normal saline 3 times and counted after adding an appropriate amount of culture medium.
(2) Primary culture of bone marrow mesenchymal stem cells: the bone marrow mesenchymal stem cells obtained in step (1) were seeded at a density of 0.3×10⁶ cells/mL in different mesenchymal stem cell culture media (which are composed of basal culture medium and medium additives, and have specific components shown in Table 1), respectively, and cultured for 3 days. Then, the number of bone marrow mesenchymal stem cells cultured in each group of culture media was measured. The results are shown in Table 1.

**Table 1**

| Group | Basal culture medium | Medium additives | | | Initial cell number (10⁶) | Cell number after 3 days (10⁶) |
|---|---|---|---|---|---|---|
| | | NMN (µM) | Melatonin (µM) | Coenzyme Q10 (µM) | | |
| Group 1 | Serum-free Mesenchymal Stem Cell Medium | 1 | 1 | 1 | 0.3 | 1.65 |
| Group 2 | Serum-free Mesenchymal Stem Cell Medium | 0 | 1 | 1 | 0.3 | 1.58 |
| Group 3 | Serum-free Mesenchymal Stem Cell Medium | 0 | 0 | 0 | 0.3 | 1.2 |
| Group 4 | Serum-free Mesenchymal Stem Cell Medium | 0.5 | 0 | 0 | 0.3 | 1.21 |
| Group 5 | Serum-free Mesenchymal Stem Cell Medium | 0 | 1 | 0 | 0.3 | 1.27 |
| Group 6 | Serum-free Mesenchymal Stem Cell Medium | 0 | 0 | 1 | 0.3 | 1.29 |
| Group 7 | Serum-free Mesenchymal Stem Cell Medium | 0.5 | 5 | 1 | 0.3 | 1.51 |
| Group 8 | Serum-free Mesenchymal Stem Cell Medium | 0.5 | 1 | 1 | 0.3 | 1.53 |
| Group 9 | Serum-free Mesenchymal Stem Cell Medium | 0.5 | 20 | 5 | 0.3 | 1.44 |
| Group 10 | Serum-free Mesenchymal Stem Cell Medium | 5 | 1 | 1 | 0.3 | 1.60 |
| Group 11 | Serum-free Mesenchymal Stem Cell Medium | 10 | 1 | 10 | 0.3 | 1.39 |
| Group 12 | Serum-free Mesenchymal Stem Cell Medium | 10 | 10 | 20 | 0.3 | 1.42 |
| Group 13 | Serum-free Mesenchymal Stem Cell Medium | 20 | 50 | 50 | 0.3 | 0.4 |
| Group 14 | Serum-free Mesenchymal Stem Cell Medium | 0 | 50 | 50 | 0.3 | 0.46 |

It can be seen from Table 1 that addition of coenzyme Q10 and melatonin to the basal culture medium with a final concentration in the range from 1 µM to 20 µM in the medium and in a molar ratio ranging from 1:0.2 to 1:10, can promote the proliferation of bone marrow mesenchymal stem cells.

### Example 2

(1) Isolation of bone marrow mesenchymal stem cells: 15 mL of human bone marrow was extracted and treated with 100 U heparin per milliliter of bone marrow for anticoagulation. The normal saline was added in an equal volume to and blended with the bone marrow, and then the diluted bone marrow was slowly added onto the surface of the lymphocyte isolation liquid (Ficoll isolation liquid) in a ratio of 1 part of Ficoll isolation liquid to 2 parts of the diluted bone marrow. The resultant mixture was centrifuged in a horizontal centrifuge at 2000 r/min at 20°C for 30 minutes, and the bone marrow mesenchymal stem cells removed from the interface were washed with normal saline 3 times and counted after adding an appropriate amount of culture medium.
(2) Primary culture of bone marrow mesenchymal stem cells: the bone marrow mesenchymal stem cells obtained in step (1) were seeded at a density of 0.3×10⁶ cells/mL in different mesenchymal stem cell culture media, respectively, wherein the mesenchymal stem cell culture medium used in Group 1 was composed of the serum-free mesenchymal stem cell culture medium, 1µM of coenzyme Q10, 1µM of melatonin and 1µM of nicotinamide mononucleotide; the mesenchymal stem cell culture medium used in Group 2 was composed of the serum-free mesenchymal stem cell culture medium, 1µM of coenzyme Q10 and 1µM of melatonin; and the mesenchymal stem cell culture medium used in Group 3 was the serum-free mesenchymal stem cell culture medium alone (i.e. control group). After 3 days, the medium in each group was replaced with a corresponding fresh medium to remove non-adherent cells. Thereafter, half of the spent medium was replaced with the fresh medium every 3 days. The bone marrow mesenchymal stem cells obtained by primary culture were denoted as P0.
(3) subculture of bone marrow mesenchymal stem cells: each group of the bone marrow mesenchymal stem cells was subcultured once the cells reached 90% confluence: after the mesenchymal stem cell culture medium was pipetted completely, the cells were washed twice with normal saline. 0.25% (2.5g/L, 0.25%) trypsin preheated at 37°C (containing 1mmol/L EDTA, that is, prepared with 1mmol/L EDTA-Na2) was added to the cells for digestion for 1-2 min. The cells were observed under an inverted microscope. Once the bone marrow mesenchymal stem cells shrank, the modified mesenchymal stem cell culture medium (approximately 3 mL) was added to stop the digestion by trypsin. After repeatedly pipetting up and down, the cells were collected in a 15mL centrifuge tube and centrifuged at 1500r/min for 10 min. After the supernatant was discarded, the cells are washed twice with normal saline solution to thoroughly wash away the remaining trypsin. The cells were resuspended in the mesenchymal stem cell culture medium, seeded at a density of 5×10⁵ cells/mL in corresponding fresh mesenchymal stem cell culture medium and subcultured. The bone marrow mesenchymal stem cells obtained by the first-time subculture of primary cells were denoted as P1. Similarly, by continuous subculture, bone marrow mesenchymal stem cells from passages P2 to P9 were harvested for each group.

It should be noted that the prepared P2-P9 bone marrow mesenchymal stem cells in each group were all qualified according to the following standard of quality control method: 1) pathogen detection: before aspirating bone marrow, 2 tubes (about 2mL each) of human venous blood were drawn into a coagulation tube for pathogen detection. No Hepatitis B virus, hepatitis C virus, syphilis virus, HIV, CMV, EBV, HTLV should be detected. 2) flow cytometry: before subculture, the P2 bone marrow mesenchymal stem cells were tested for the following surface markers: CD105, which must not be less than 90%; CD73 must not be less than 90%; CD90, which must not be less than 90%; CD45, which must not exceed 2%; CD19, which must not exceed 2%; CD HLA-DR, which must not exceed 2%; CD34, which must not exceed 2%; and CD11b, which must not exceed 2%.

### Testing

### 1. Detection of cell survival rate

The P2 and P9 bone marrow mesenchymal stem cells in each group obtained in Example 2 (after subculture for 3 days) were tested: Trypan blue staining was used to detect the cell survival status of P2 and P9 bone marrow mesenchymal stem cells in each group, and the cell survival rate was calculated. The results of the cell survival rate of each group are shown in Table 2.

**Table 2**

| Group | Cell survival rate | |
|---|---|---|
| | P2 | P9 |
| Control Group | 99.50% | 99.10% |
| Group 1 | 99.52% | 99.08% |
| Group 2 | 99.48% | 99.01% |

As shown in Table 2, the cell survival rates of the P2 bone marrow mesenchymal stem cells in the control group, group 1 and group 2 were all above 98%, and the cell survival rate of the P9 bone marrow mesenchymal stem cells in the control group, group 1 and group 2 were all above 98%.

### 2. Flow cytometric detection of cell surface markers

The three groups of P2 bone marrow mesenchymal stem cells prepared in Example 2 were respectively labeled using a Human Mesenchymal Stem Cell Detection kit (BD, #562245) for detection of surface markers by flow cytometer (BD Accuri C6). The results are shown in Table 3.

**Table 3**

| Cell Surface Markers | Control Group | Group 1 | Group 2 | Standard |
|---|---|---|---|---|
| CD90 | 97.90% | 98.40% | 99.00% | ≥95% |
| CD105 | 99.10% | 99.60% | 98.30% | ≥95% |
| CD73 | 99.20% | 98.50% | 98.20% | ≥95% |
| CD44 | 98.70% | 98.20% | 99.00% | ≥95% |
| CD34/CD11b/CD19/CD45/HLA-DR | 1.30% | 1.40% | 1.20% | ≤2% |

As shown in Table 3, the P2 bone marrow mesenchymal stem cells in the control group, group 1, and group 2 all met the standards for mesenchymal stem cell surface markers.

### 3. Testing of mitochondrial membrane potential in bone marrow mesenchymal stem cells

The P2 and P9 bone marrow mesenchymal stem cells in each group obtained in Example 2 (after subculture for 3 days) were tested for membrane potential: after labeling with TMRE (tetramethylrhodamine ethyl ester), the numbers of TMRE-positive cells were measured by flow cytometry and analyzed for significance versus the control group. The experiment was performed in triplicate. The results are shown in Table 4.

**Table 4**

| Control Group | | | Group 1 | | | Group 2 | | |
|---|---|---|---|---|---|---|---|---|
| Passage | Number of TMRE-positive Cells | SEM | Number of TMRE-positive Cells | SEM | Significance | Number of TMRE-positive Cells | SEM | Significance |
| P2 | 43.85% | 2.84% | 56.92% | 3.27% | Yes | 57.93% | 4.18% | Yes |
| P9 | 23.19% | 3.38% | 48.65% | 3.90% | Yes | 52.17% | 3.86% | Yes |

As shown in Table 4, the number of TMRE-positive cells gradually decreased as bone marrow mesenchymal stem cells were passaged more times. The numbers of TMRE-positive cells in P2 bone marrow mesenchymal stem cells in both Group 1 and Group 2 were both higher than and significantly different from those in the control group; and the numbers of TMRE-positive cells in P9 bone marrow mesenchymal stem cells in both Group 1 and Group 2 were both higher than and significantly different from those in the control group . Therefore, the media used in Group 1 and Group 2 have a protective effect on the mitochondrial membrane potential in the bone marrow mesenchymal stem cells cultured therein, and can slow down the reduction of mitochondrial activity in the bone marrow mesenchymal stem cells caused by subculture.

### 4. Test of mitochondrial superoxygen compounds in bone marrow mesenchymal stem cells

The P2 and P9 bone marrow mesenchymal stem cells in each group obtained in Example 2 were tested for mitochondrial superoxygen compounds: after labeling with MitoSOX (Intracellular oxidative stress reactive oxygen species superoxide anion), the numbers of MitoSOX-positive cells were measured by flow cytometry and analyzed for significance versus the control group. The experiment was performed in triplicate. The results are shown in Table 5.

**Table 5**

| Control Group | | | Group 1 | | | Group 2 | | |
|---|---|---|---|---|---|---|---|---|
| Passage | Number of MitoSOX - positive Cells | SEM | Number of MitoSOX - positive Cells | SEM | Significance | Number of MitoSOX - positive Cells | SEM | Significance |
| P2 | 13.95% | 3.36% | 9.23% | 3.86% | Yes | 9.81% | 3.24% | Yes |
| P9 | 26.16% | 4.28% | 12.39% | 3.29% | Yes | 11.12% | 3.20% | Yes |

As shown in Table 5, the number of MitoSOX-positive cells gradually increased as bone marrow mesenchymal stem cells were passaged more times. The numbers of MitoSOX-positive cells in P2 bone marrow mesenchymal stem cells in both Group 1 and Group 2 were both lower than and significantly different from those in the control group; and the numbers of MitoSOX-positive cells in P9 bone marrow mesenchymal stem cells in both Group 1 and Group 2 were both lower than and significantly different from those in the control group with a significant difference. Therefore, the media used in Group 1 and Group 2 can increase the mitochondrial activity in the bone marrow mesenchymal stem cells cultured therein.

### 5. Measurement of relative number of mitochondria

Mitochondria in cells were measured for the relative number by amplifying the ND1 gene in mtDNA using qPCR method with the following related primers: ND1-F: 5'-ATACAACTACGCAAAGGCCCCA-3' (SEQ ID No.1), ND1-R: 5'-AATAGGAGGCCTAGGTTGAGGT-3' (SEQ ID No.2); β-actin-F: 5'-CGGGAAATCGTGCGTGACAT-3' (SEQ ID No.3), β-actin-R: 5'-GAAGGAAGGCTGGAAGAGTG-3' (SEQ ID No.4). The experiment was performed in a semi-quantitative manner using 2x Trans Green QPCR Mix (Abgent) in a qPCR instrument (Qiagen). The mitochondria in P2 control group were counted as a relative value of 1. The experiment was performed in triplicate. The results are shown in Table 4.

**Table 6**

| Control Group | | | Group 1 | | | Group 2 | | |
|---|---|---|---|---|---|---|---|---|
| Passage | Relative number of mitochondria | SEM | Relative number of mitochondria | SEM | Significance | Relative number of mitochondria | SEM | Significance |
| P2 | 1 | 0.12 | 1.24 | 0.15 | Yes | 1.36 | 0.10 | Yes |
| P9 | 0.87 | 0.24 | 1.13 | 0.27 | Yes | 1.27 | 0.31 | Yes |

As shown in Table 6, the number of mitochondria shows a downward trend as bone marrow mesenchymal stem cells were passaged more times. The numbers of MitoSOX-positive cells in P2 bone marrow mesenchymal stem cells in both Group 1 and Group 2 were both higher than and significantly different from those in the control group; and the numbers of MitoSOX-positive cells in P9 bone marrow mesenchymal stem cells in both Group 1 and Group 2 were both higher than and significantly different from those in the control group. Therefore, the media used in Group 1 and Group 2 can increase the number of mitochondria in the bone marrow mesenchymal stem cells cultured therein.

In summary, the mesenchymal stem cell culture medium composed of the serum-free mesenchymal stem cell culture medium, 1µM of coenzyme Q10, 1µM of melatonin and 1µM of nicotinamide mononucleotide and the mesenchymal stem cell culture composed of the serum-free mesenchymal stem cell culture medium, 1µM of coenzyme Q10, and 1µM of melatonin can enhance the mitochondrial activity of bone marrow mesenchymal stem cells and increase the number of mitochondria.

### Example 3

### Effect of the bone marrow mesenchymal stem cells prepared in Example 2 in the treatment of type 2 diabetes

P3 bone marrow mesenchymal stem cells (also called "enhanced bone marrow mesenchymal stem cells", hereinafter referred to as eBMSCs) cultured in the mesenchymal stem cell culture medium, which is composed of the serum-free mesenchymal stem cell culture medium, 1µM of coenzyme Q10, 1µM of melatonin and 1µM of nicotinamide mononucleotide, in Example 2, were used in preclinical trials in type 2 diabetic mice. specifically,
(1) 8-week-old male C57BL/6 mice were divided into two groups. One group of mice (n=12) was fed with a high-fat diet containing 60% of fat and denoted as high-fat diet (HFD) group, while the other group (n=6) was fed with a normal mouse maintenance diet and denoted as normal diet (ND) group. Table 7 shows the statistical results of the changes in body weight of mice of the two groups over time.

**Table 7**

| Weeks | **ND Group** | | **HFD Group** | | |
|---|---|---|---|---|---|
| | **Weight/g** | **SEM** | **Weight/g** | **SEM** | **Significance** |
| **0** | 18.2 | 0.8 | 18.1 | 0.7 | No |
| **4** | 19.5 | 0.7 | 19.4 | 0.9 | No |
| **6** | 20.3 | 0.9 | 20.7 | 1.2 | No |
| **8** | 21 | 0.4 | 22.9 | 1.4 | No |
| **10** | 23.1 | 0.8 | 26.4 | 1.2 | Yes |
| **12** | 23.6 | 1.1 | 30.3 | 1.1 | Yes |
| **14** | 24.3 | 0.9 | 33.5 | 1.5 | Yes |
| **16** | 25.2 | 1 | 36.9 | 1.3 | Yes |
| **18** | 26.9 | 1.1 | 38.2 | 0.9 | Yes |
| **20** | 27.6 | 1.3 | 39.7 | 1.6 | Yes |
| **22** | 28.2 | 1 | 40.3 | 2.1 | Yes |
| **24** | 28.4 | 1.2 | 41.9 | 2.5 | Yes |
| **26** | 28.5 | 1.4 | 45.3 | 2.1 | Yes |
| **28** | 28.9 | 0.8 | 50.1 | 2.6 | Yes |
| **30** | 29.3 | 1.5 | 53.3 | 2.9 | Yes |

As shown in Table 7, the experimental group (HFD group) was significantly different from the control group (ND group) in weight from week 10 after modeling. Further, statistical analysis showed that there was a very significant difference between the HFD group and the ND group from week 14.
(2) According to the diagnostic criteria for type 2 diabetic mice set by the WHO in 2016, a diet-induced type 2 diabetes model mouse is a mouse having a body weight heavier than 40 g for 4 consecutive weeks, a fasting blood glucose level higher than 6.1mM, and a blood glucose level at 2 hours after intraperitoneal injection of glucose higher than 11.1mM. Therefore, a glucose tolerance test was performed on mice from week 28 after modeling: after starvation for 16 hours, glucose was intraperitoneally injected at 1 g/kg body weight, and blood glucose was tested. The results are shown in Table 8.

**Table 8**

| Time from glucose injection after fasting (min) | ND Group | | HFD Group | | |
|---|---|---|---|---|---|
| | Mean of blood glucose (mM) | SEM | Mean of blood glucose (mM) | SEM | Significance |
| 0 | 5.3 | 0.5 | 8.8 | 0.7 | Yes |
| 15 | 13.6 | 0.9 | 23.9 | 1.2 | Yes |
| 30 | 12.5 | 0.6 | 27.4 | 2.1 | Yes |
| 60 | 9.8 | 0.4 | 25.5 | 2.8 | Yes |
| 120 | 6.5 | 0.8 | 19.4 | 2.4 | Yes |

As shown in Table 8, in week 28, the mice in HFD group had a body weight heavier than 40 g for 4 consecutive weeks and a mean blood glucose level higher than 6.1mM, which are significantly different from those of the ND group. After 120 minutes from glucose injection after fasting, the mean blood glucose level was 19.4mM, higher than 11.1mM. Thus, the diet-induced type 2 diabetes mouse model was successfully constructed.
In addition, the serum insulin content in mice in HFD and ND groups after 5 hours of fasting was tested. The results are shown in Table 9.

**Table 9**

| Serum insulin in ND group | | Serum insulin in HFD group | | |
|---|---|---|---|---|
| Mean (ng/mL) | SEM | Mean (ng/mL) | SEM | Significance |
| 0.74 | 0.04 | 10.6 | 3.25 | Yes |

As shown in Table 9, the serum insulin level in mice in the HFD group was significantly higher than that of the control group, which, in combination with results of the significant increase in blood sugar in the HFD group after starvation, indicates that the HFD group had an obvious symptom of type 2 diabetes, that is, insulin resistance.
In addition, an insulin tolerance test was conducted: at 4 hours after starvation, insulin was injected intraperitoneally at 1U/kg body weight. The results are shown in Table 10.

**Table 10**

| | **ND Group** | | **HFD Group** | | |
|---|---|---|---|---|---|
| Time from insulin injection (min) | Mean of blood glucose (min) | SEM | Mean of blood glucose (min) | SEM | Significance |
| 0 | 8.5 | 0.4 | 13.4 | 0.8 | Yes |
| 15 | 5.4 | 0.7 | 12.7 | 1 | Yes |
| 30 | 5.3 | 0.9 | 10.1 | 1.4 | Yes |
| 60 | 5 | 1.1 | 9.8 | 1.9 | Yes |
| 120 | 4.8 | 1.5 | 9.6 | 1.6 | Yes |

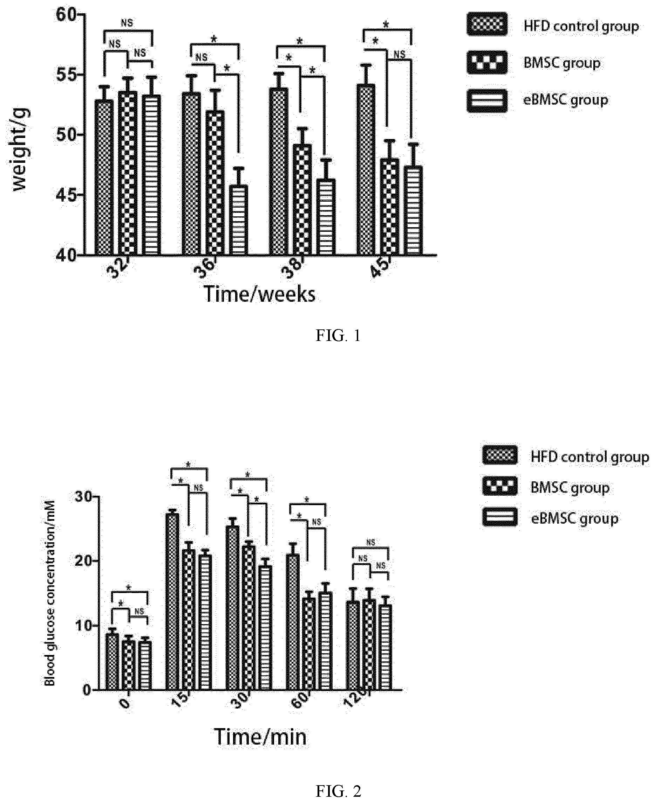

As shown in Table 10, after insulin injection, the blood glucose in mice of the HFD group did not decrease by the same extent as that of the ND group mice, which further verified that the HFD group showed an insulin resistance symptom, indicating a successful constructed type 2 diabetes model.
(3) The mice in the HFD group were kept on the high-fat diet. At weeks 32 and 34 (from the beginning of feeding with the high-fat diet), mice successfully modeled in the HFD group were each administered by injection into the tail vein, specifically administered in the following groups:
HFD control group, which was injected with normal saline; BMSC group, which was injected with P3 bone marrow mesenchymal stem cells cultured in the serum-free mesenchymal stem cell culture medium at 5×10⁶ cells/mice; and eBMSC group, which was injected with eBMSCs at 5×10⁶ cells/mice. The mice were weighed during the continued feeding. The results are shown in Table 11 and FIG. 1.

**Table 11**

| Week | HFD Control Group | | | BMSC Group | | eBMSC Group | | |
|---|---|---|---|---|---|---|---|---|
| | Weight(g) | SEM | Weight(g) | SEM | Significance | Weight(g) | SEM | Significance |
| 32 | 52.8 | 1.2 | 53.5 | 1.2 | No | 53.2 | 1.6 | No |
| 36 | 53.4 | 1.5 | 51.9 | 1.8 | No | 45.7 | 1.5 | Yes |
| 38 | 53.8 | 1.3 | 49.1 | 1.4 | Yes | 46.2 | 1.7 | Yes |
| 45 | 54.1 | 1.7 | 47.9 | 1.6 | Yes | 47.3 | 1.9 | Yes |

As shown in table 1 and FIG.1, compared with the HFD control group, the weight of the mice significantly decreased within two weeks in the BMSC group and significantly decreased within one week in the eBMSC group, indicating that the bone marrow mesenchymal stem cells have a significant weight loss effect. Further, statistical analysis showed that the effect in the eBMSC group was significantly better than that in the BMSC group and could last at least 11 weeks in mice.
In addition, the glucose tolerance test was performed in week 36 (that is, at 2 weeks after the last medicament injection). The results are shown in Table 12 and FIG. 2.

**Table 12**

| | **HFD Control Group** | | **BMSC Group** | | | **eBMSC Group** | | |
|---|---|---|---|---|---|---|---|---|
| Time from glucose injection after fasting for 16 h (min) | Mean of blood glucose (min) | SEM | Mean of blood glucose (min) | SEM | Significance | Mean of blood glucose (min) | SEM | Significance |
| 0 | 8.6 | 0.9 | 7.5 | 0.9 | Yes | 7.4 | 0.7 | Yes |
| 15 | 27.2 | 0.7 | 21.6 | 1.3 | Yes | 20.8 | 0.9 | Yes |
| 30 | 25.3 | 1.3 | 22.2 | 0.8 | Yes | 19.1 | 1.2 | Yes |
| 60 | 20.9 | 1.8 | 14.1 | 1.1 | Yes | 15.0 | 1.5 | Yes |
| 120 | 13.6 | 2.1 | 13.9 | 1.8 | No | 13.1 | 1.3 | No |

As shown in Table 12 and FIG. 2, the BMSC group had a significant hypoglycemic effect compared with the HFD control group. The hypoglycemic effect of the eBMSC group was better, and significantly better than that of the BMSC group.

### Example 4

Effect of the bone marrow mesenchymal stem cells prepared in Example 2 on fatty liver:
The mice in the ND, HFD control, BMSC, and eBMSC groups in Example 3 were kept on the corresponding diet (normal diet for ND; high-fat diet for HFD control, BMSCs, and eBMSCs) until being sacrificed at week 46. The liver tissues of the mice were respectively prepared into paraffin sections, and stained with hematoxylin, specifically following the procedure as below:
(1) Preparation of tissue paraffin sections:
   a. the mouse was injected with 5% chloral hydrate (100 µL/10 g body weight), and after the mouse was anesthetized, its chest cavity was quickly opened, and the liver is taken out and placed in PBS solution;
   b. the liver tissue after adipose tissue being removed with microscissors was placed in 4% paraformaldehyde in PBS solution, and immobilized for 48 hours in a shaker at 4°C;
   c. the immobilized liver tissue was placed into 75% ethanol solution for dehydration;
   d. the dehydrated tissue was placed in a gradient of xylene solutions.
   e. the tissue was placed in a paraffin embedding machine (Leica) with a thermal console at 60°C and an iron groove in the depth of 6 mm for embedding, specifically, the tissue was placed in the groove, into which paraffin wax was dropped, and the tissue was placed on a cooling console for molding;
   f. the tissue was cut into 5 µm thick slices using a paraffin microtome (Leica), the slices were then stretched in water at 45°C, placed on the anti-off glass slide, and dried in an oven at 60°C for 5 min for next use.
(2) H&E staining:
   a. H&E staining reagents: hematoxylin formula: 2 g of hematoxylin, 40 mL of absolute ethanol, 100 g of aluminum potassium sulfate, 600 mL of distilled water, 0.4 g of sodium iodate, and 20 mL of glacial acetic acid.
   b. formulation method: distilled water was first added followed by potassium aluminum sulfate, and after heating slightly, hematoxylin, absolute ethanol, sodium iodate, and glacial acetic acid were added for sufficient dissolution; eosin solution formula: 2.5 g of eosin (alcohol-soluble), 1 L of 95% ethanol; formulation method of glacial acetic acid: eosin was dissolved in 95% ethanol, stirred, and adjusted to pH 4.5 with glacial acetic acid; hydrochloric acid ethanol differentiation solution: 1 mL of concentrated hydrochloric acid, and 200 mL of 75% ethanol.
   c. staining step:
      Conventional dewaxing: the paraffin sections were dried in an oven at 60°C for 15 minutes, soaked in xylene 3 times with each time for 10 minutes, and soaked successively in 100% ethanol, 100% ethanol, 95% ethanol, 95% ethanol, 90% ethanol, 90% ethanol, 85% ethanol 75% ethanol for 5 min each.

Staining: the paraffin sections were soaked in hematoxylin for 5 minutes, washed in tap water, differentiated in hydrochloric acid and ethanol for 30 sec each, washed in tap water again, soaked in eosin solution for 5 minutes, and washed in tap water again.

Dehydration: the paraffin sections were taken out quickly from 95% ethanol, soaked in 100% ethanol 3 times with each time for 10 sec, in xylene 3 times with each time for 1.5 min, and mounted using neutral balsam with a coverslip in thickness ranging from 0.13 mm to 0.17 mm.

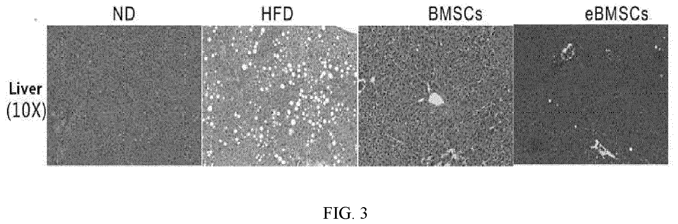

The paraffin sections in each group after staining are shown in FIG. 3. As shown in FIG. 1, the mice in the BMSC group showed a significantly reduced fatty liver, with an effective rate of about 80%, and the mice in the eBMSC group also showed a significantly reduced fatty liver, with an effective rate of 100%. Therefore, the bone marrow mesenchymal stem cells have a significant therapeutic effect on the fatty liver.

The various technical features of the above-mentioned embodiments can be combined arbitrarily. To make the description concise, all possible combinations of the various technical features in the above-mentioned embodiments are not described. However, the combinations of these technical features should be regarded as the scope of this specification as long as these technical features have no collision with each other.

The above-mentioned embodiments only express several embodiments of the present disclosure with more specific and detailed descriptions, but should not be understood as limiting the scope of the disclosure. It should be noted that for those of ordinary skill in the art, several modifications and improvements can be made without departing from the concept of the present disclosure and these all fall within the protection scope of the present disclosure. Therefore, the protection scope of the present disclosure should be subject to the appended claims.

## Claims

1. A modified mesenchymal stem cell culture medium, comprising a basal culture medium, a first component, and melatonin, wherein the first component is at least one selected from the group consisting of coenzyme Q10 and mitoquinone mesylate, and has a working concentration in a range from 1 µM to 20 µM, the melatonin has a working concentration in a range from 1 µM to 20 µM, and the first component and the melatonin are in a molar ratio ranging from 1:0.2 to 1:10.

2. The modified mesenchymal stem cell culture medium according to claim 1, wherein the modified mesenchymal stem cell culture medium further comprises a second component, and the second component is at least one selected from the group consisting of nicotinamide mononucleotide, nicotinamide ribose, and NADH.

3. The modified mesenchymal stem cell culture medium according to claim 2, wherein the first component and the second component are in a molar ratio ranging from 1:0.2 to 1:10.

4. The modified mesenchymal stem cell culture medium according to claim 1, wherein the modified mesenchymal stem cell culture medium further comprises a second component, the second component is nicotinamide mononucleotide, the first component is coenzyme Q10, and the working concentration of the first component is in a range from 1 µM to 5 µM, the working concentration of the melatonin is in a range from 1 µM to 5 µM, and the working concentration of the second component is in a range from 0.5 µM to 10 µM.

5. The modified mesenchymal stem cell culture medium according to any one of claims 1 to 4, wherein the basal culture medium is one selected from DMEM, EMEM, IMDM, GMEM, RPMI-1640, and α-MEM.

6. The modified mesenchymal stem cell culture medium according to claim 5, wherein the modified mesenchymal stem cell culture medium further comprises at least one of vitamin B, minerals, polyphenols, L-carnitine, alpha lipoic acid, pyrroloquinoline quinone, and creatine.

7. A method for culturing bone marrow mesenchymal stem cells comprising the following step:
culturing the bone marrow mesenchymal stem cells in the modified mesenchymal stem cell culture medium according to any one of claims 1 to 6.

8. The method for culturing bone marrow mesenchymal stem cells according to claim 7, wherein the bone marrow mesenchymal stem cells are human bone marrow mesenchymal stem cells.

9. A bone marrow mesenchymal stem cell obtained by the method for culturing bone marrow mesenchymal stem cells according to claim 7 or 8.

10. The bone marrow mesenchymal stem cell according to claim 9 for use in the treatment of type 2 diabetes or fatty liver.

11. A medicament for the treatment of type 2 diabetes or fatty liver comprising the bone marrow mesenchymal stem cells according to claim 10.
